# EUROPEAN PATENT APPLICATION

(11) **EP 2 626 004 A1**
(43) Date of publication of application: **14.08.2013**
(21) Application number: 12167687.8
(22) Date of filing: 11.05.2012
(51) Int. Cl.: A61B 3/13

(54) **Non-visible light ophthalmic biomicroscope**

(30) Priority: 09.02.2012 ES 201230193
(71) Applicant: Amedmore, S.L., 15706 Santiago de Compostela (ES)
(72) Inventor: Moreno Manresa, Javier Gregorio, 15706 SANTIAGO DE COMPOSTELA (ES)
(74) Representative: Lahidalga de Careaga, Jose Luis

(57) **Abstract**

The invention relates to a non-visible light ophthalmic biomicroscope essentially characterized in that the invention is made up at least of the following elements:
.-a very low intensity visible spectrum and/ non-visible spectrum light emitter (2) creating a light field (3) which is located between the eye of the patient (1) to be examined and the receptive cameras (5) and (6).
.- respective receptive cameras (5) and (6) for the eyes (LE)and (RE) of the observer.
.- an image processing device (7) for the digital processing of all the data received by the receptive cameras (5) and (6).
.- respective digital viewers for the eyes (LE) and (RE) of the observer.

## Description

### Object of the Invention

The invention proposed relates to a non-visible light ophthalmic biomicroscope particularly suitable for examining patients where photophobia is a constant symptom.

### Field of the Invention

The field of the invention is that of manufacturing electronic devices and auxiliary means for medical diagnostics.

The field of the invention also relates to the ophthalmological sector, particularly diagnostic sector.

### Background of the Invention

The toxic effect of light on photoreceptors is widely known and has been a concern for ophthalmologists for many years, particularly with respect to the potential damage which can be caused by the light of surgical microscopes and the endoillumination sources used in vitrectomy surgeries.

On the other hand patient discomfort while being evaluated under the slit lamp with a high light intensity, particularly in terms of that relating to funduscopy under pupil dilation have been verified by all ophthalmologists.

There are some prior arts for the system described in this specification.

For example, the following publications can be referred to in terms of the toxic effects of light.

And at least the following publications are considered as the prior arts in terms of the endoillumination sources.

With respect to the prior art, the present invention involves at least the following improved features:
a.- a much more positive replacement for the traditional slit lamp (Gullstrand 1911).
b.- it can allow freeing one of the surgeon's hands in the case of vitrectomy surgery.
c.- the patient is comfortable since he/she does not have to be subjected to visible light and especially in those patients whose eyes have some sort of inflammation where photophobia is a constant symptom.

The inventor is unaware of any prior art incorporating the arrangements presented by this invention, or the advantages entailed by said arrangement.

### Description of the Invention

The non-visible light ophthalmic biomicroscope proposed by the invention is made up at least of the following elements:
.- non-visible spectrum light emitter creating a light field which is located between the eyes of the patient (RE)(1) and (LE)(2)and an optical device
.- This optical device has different magnifications, similar to those used in traditional examinations with slit lamps.
.- receptive cameras for the eyes (LE)and (RE).

They are heat-sensitive video cameras which are sensitive to non-visible spectrum light.
.- image processing device. This device digitally processes all the data received by the receptive cameras.
.- digital viewer of the left eye (10)
.- digital viewer of the right eye (11).

The two viewers allow the observer to better evaluate the images obtained during the examination since the observer has a depth view due to the double reception (right eye (RE) and left eye (LE)).

Furthermore, in comparison with the traditional methods, more information can be obtained with the aid of the digital filters incorporated in the image processing device.

### Description of the Drawings

To better understand the invention, a set of drawings are attached in which the following is seen.

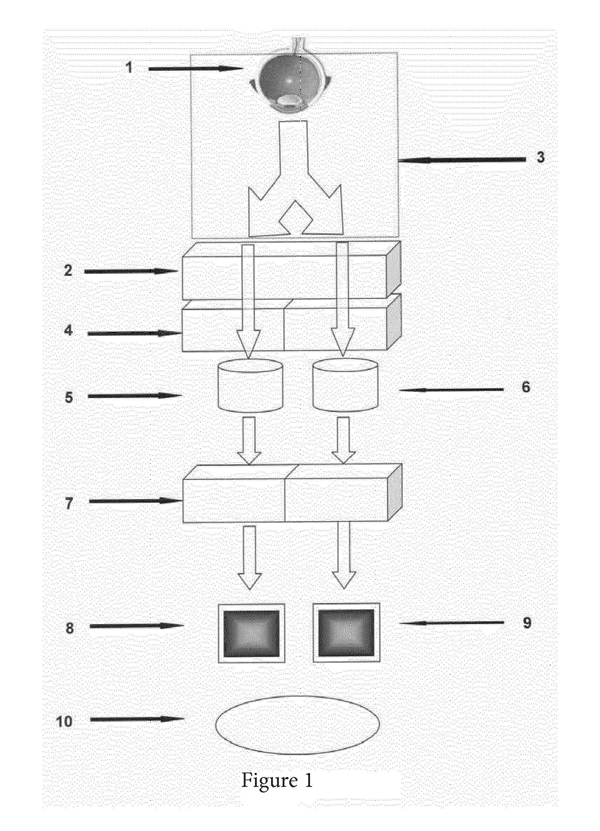

Figure 1 shows a diagram of the components of the non-visible light ophthalmological biomicroscope

Identical elements are designated in said drawings with the same reference, among which include:
1.- eye to be observed
2.- very low intensity visible spectrum and/or non-visible spectrum light emitter
3.- light field of the emitter (3).
4.- optical device.
5.- receptive camera (LE)
6.- receptive camera (RE).
7.- image processing device.
8.- digital viewer for the left eye of the observer (LE)
9.- digital viewer for the right eye of the observer (RE).
10.- observer

### Preferred Embodiment of the Invention

The non-visible light ophthalmic biomicroscope proposed by the invention is made up at least of the following elements:
.- very low intensity visible spectrum and/or non-visible spectrum light emitter ()creating a light field (3) which is located between the eye of the patient (1) to be examined and the receptive cameras (5) and (6).

They are video cameras which are sensitive to very low intensity visible spectrum and/or non-visible spectrum light and/or are heat-sensitive.
.- optical device (4). This optical system has different magnifications, similar to those used in traditional examinations with slit lamps.
.- receptive cameras (5) and (6) for the eyes (LE) and (RE) of the observer. They are heat-sensitive video cameras which are sensitive to very low intensity visible spectrum and/or non-visible spectrum light and/or heat-sensitive.
.- image processing device (7). This device digitally processes all the data received by the receptive cameras (5) and (6).
.- digital viewer for the left eye (8) of the observer.
.- digital viewer of the right eye (9) of the observer.

Both viewers allow the observer to evaluate the images obtained during the examination with a depth view due to the simultaneous double reception for the right eye (RE) and left eye (LE) of the observer.

Furthermore in comparison with the traditional methods, more information can be obtained with the aid of the digital filters incorporated in the image processing device (7).

Having sufficiently described the nature of the invention as well as a way of carrying it out to practice, it merely remains to be said that arrangements indicated above and depicted in the attached drawings are susceptible to detail modifications as long as the their essential principles established in the paragraphs above and summarized in the following claims are not altered.

## Claims

1. A non-visible light ophthalmic biomicroscope essentially **characterized in that** it is made up at least of the following elements:
.- non-visible spectrum light emitter (3) creating a light field (3) which is located between the eye of the patient (1) to be examined and the receptive cameras (5) and (6).
.- optical device (4) further having the possibility of incorporating different magnifications.
.- receptive cameras (5) and (6) for the eyes (LE) and (RE) of the observer.
.- image processing device (7) for the digital processing of all the data received by the receptive cameras (6) and (7).
.- digital viewer for the left eye (8) of the observer
.- digital viewer for the right eye (9) of the observer.

2. The non-visible light ophthalmic biomicroscope according to claim 1, **characterized in that** the light spectrum can be the very low intensity visible spectrum and/or non-visible spectrum.

3. The non-visible light ophthalmic biomicroscope according to the preceding claims, **characterized in that** the receptive cameras (5) and (6) are heat-sensitive video cameras which are sensitive to very low intensity visible spectrum and/or non-visible spectrum light and/or are heat-sensitive.
